(19) **Europäisches Patentamt / European Patent Office / Office européen des brevets**

(11) **EP 2 901 913 A1**

(12) # EUROPEAN PATENT APPLICATION
published in accordance with Art. 153(4) EPC

(43) Date of publication:
**05.08.2015 Bulletin 2015/32**

(51) Int Cl.:
**A61B 1/00** *(2006.01)*　　**G02B 23/24** *(2006.01)*

(21) Application number: **13842411.4**

(86) International application number:
**PCT/JP2013/067543**

(22) Date of filing: **26.06.2013**

(87) International publication number:
**WO 2014/050236 (03.04.2014 Gazette 2014/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.09.2012　JP 2012216301**

(71) Applicant: **Olympus Medical Systems Corp.**
**Tokyo 151-0072 (JP)**

(72) Inventor: **ITO, Takashi**
**Hachioji-shi**
**Tokyo 192-8512 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **ENDOSCOPE DEVICE HAVING CLEANING MECHANISM**

(57)　In a cleaning mechanism provided in an insertion section of an endoscopic device, two straight lateral faces obliquely and straightly extending toward the center side of a cylindrical lens are joined to a laterally circumferential face, a pedestal section inside which a liquid-sending route of a cleaning liquid, and a gas-sending route are internally provided is arranged, and an angle which a gushing direction of the cleaning liquid gushed from each of wash nozzles provided on the lateral faces toward the laterally circumferential face forms with a perpendicular line passing through the center of the cylindrical lens is an acute angle, the gushed cleaning liquid intersecting the perpendicular line.

F I G. 2A

**Description**

Technical Field

The present invention relates to an endoscopic device including a direct-viewing observation port used to directly view the front, and a side-viewing observation port used to observe the lateral circumference by using a cylindrical lens which are provided at a tip of an insertion part, and provided with a cleaning mechanism configured to clean the observation ports.

Background Art

In an endoscopic device generally used, a direct-viewing observation port used to carry out imaging in the insertion direction (axial direction) in a predetermined visual field region, illumination port through which illuminating light for observation is applied, forceps hole used to extend forceps or the like, and so on are arranged on the tip face of an insertion section.

Further, in, for example, patent literature 1, an endoscopic device provided with a side-viewing observation port employing a cylindrical optical element configured to form an image of an observation object in the lateral circumference (surroundings around the axis) orthogonality to the axial direction is proposed.

For example, in the observation based on a large intestine endoscopic device, a large intestine which is the observation object has many folds, and there is the possibility of an oversight occurring unless observations are made carefully by turning the bending part. Thus, by providing a side-viewing observation port, it is possible to make the visual field region wider, and more securely prevent an oversight from occurring. Furthermore, an endoscopic device provided with a direct-viewing observation port configured to make direct-viewing observations, and side-viewing observation port configured to make side-viewing observations of the lateral circumference as shown in patent literature 2 is proposed.

Further, normally, the insertion section of the endoscope is inserted into the body cavity of a patient, and hence a foreign substance remaining in the body cavity adheres to the observation port provided at the tip thereof. Adhesion of the foreign substance to the observation port hinders the observations, and hence as proposed in, for example, patent literature 3, a nozzle is arranged in the vicinity of the observation port, and a gaseous body and/or a liquid body is gushed as the need arises, whereby the foreign substance is removed, and the observation port is cleaned.

Citation List

Patent Literature

Patent Literature 1: Jpn. Pat. Appln. KOKAI Publication No. 2010-169792
Patent Literature 2: Jpn. Pat. Appln. KOKAI Publication No. 2009-15252
Patent Literature 3: Jpn. Pat. Appln. KOKAI Publication No. 05-103752

Summary of Invention

Technical Problem

When the aforementioned direct-viewing observation port, and the side-viewing observation port are to be provided on the tip face of the insertion section, if the configuration for producing an image based on direct-viewing, and side-viewing utilizing the same imaging element is employed, a cylindrical lens structure is conceivable in which a side-viewing observation port is arranged on the tip face in the axial direction, and a direct-viewing observation port is arranged on the front side of the side-viewing observation port to overlay it. Further, a nozzle used to wash the direct-viewing observation port is required, and hence a structure configured to arrange a nozzle at a position reaching the height of the direct-viewing observation port should be formed. Furthermore, it is also necessary to arrange a nozzle used to wash the side-viewing observation port.

Solution to Problem

The present invention provides an endoscopic device including a direct-viewing observation port, and side-viewing observation port which are provided on a tip face of an insertion section, and provided with a cleaning mechanism configured to clean the observation ports.

According to an embodiment of the present invention, there is provided an endoscopic device including a cleaning mechanism characterized by comprising: a cylindrical lens arranged at a tip of an insertion section of an endoscope as an optical member for observation with a face thereof laterally circumferential with respect to an axial direction which is an insertion direction exposed to the outside; a pedestal section two lateral faces of which obliquely and straightly extending toward the central side of the cylindrical lens are joined to the laterally circumferential face, and inside which a liquid-sending route configured to send a cleaning liquid, and a gas-sending route configured to send a gaseous body are internally provided; and two wash nozzles each of which is provided on each of the two lateral faces, and is configured to selectively gush a fluid current obtained by mixing the cleaning liquid, and the gaseous body, only the liquid or only the gaseous body, an angle which gushing directions of the cleaning liquid straightly gushed from the two wash nozzles toward the laterally circumferential faces form with each other is an acute angle, the gushing directions intersecting each other, the cleaning liquid flows along the laterally circumferential face via the joint part between the pedestal section, and the laterally circumferential face.

Advantageous Effects of Invention

According to the present invention, it is possible to provide an endoscopic device in which a direct-viewing observation port, and side-viewing observation port are provided on the tip face of the insertion section, and which includes a cleaning mechanism configured to clean each of the observation ports.

Brief Description of Drawings

FIG. 1 is a view showing the external configuration of an endoscopic device provided with a cleaning mechanism configured to clean an imaging unit including a direct-viewing observation port, and side-viewing observation port according to a first embodiment.
FIG. 2A is a view showing the external configuration of an insertion section tip.
FIG. 2B is a view showing the configuration of the insertion section tip viewed from the front.
FIG. 2C is a view showing a configuration example of a wash nozzle.
FIG. 3 is a view showing a configuration example of gas-sending/water-sending pathways incorporated in the endoscopic device.
FIG. 4 is a view used to explain cleaning at a lens unit and cleaning mechanism of the embodiment based on a cleaning liquid gushed from the wash nozzles.
FIG. 5 is a view showing a configuration example of a gas-sending/liquid-sending mechanism of the embodiment.
FIG. 6 is a view showing a relationship between a distance X from a straight line passing through the lens along the central axis thereof, and angle $\beta$ between the lens central axis and water-gushing direction.
FIG. 7 is a view used to explain cleaning at a generally conceivable lens unit, and cleaning mechanism based on a cleaning liquid gushed from a wash nozzle.
FIG. 8 is a view conceptually showing the configuration of an optical system of an imaging section.
FIG. 9A is a view showing the external configuration of an insertion section tip of an endoscopic device according to a second embodiment.
FIG. 9B is a view showing the configuration of the insertion section tip viewed from the front.

Brief Description of Embodiments

Hereinafter, embodiments of the present invention will be described below in detail with reference to the drawings.

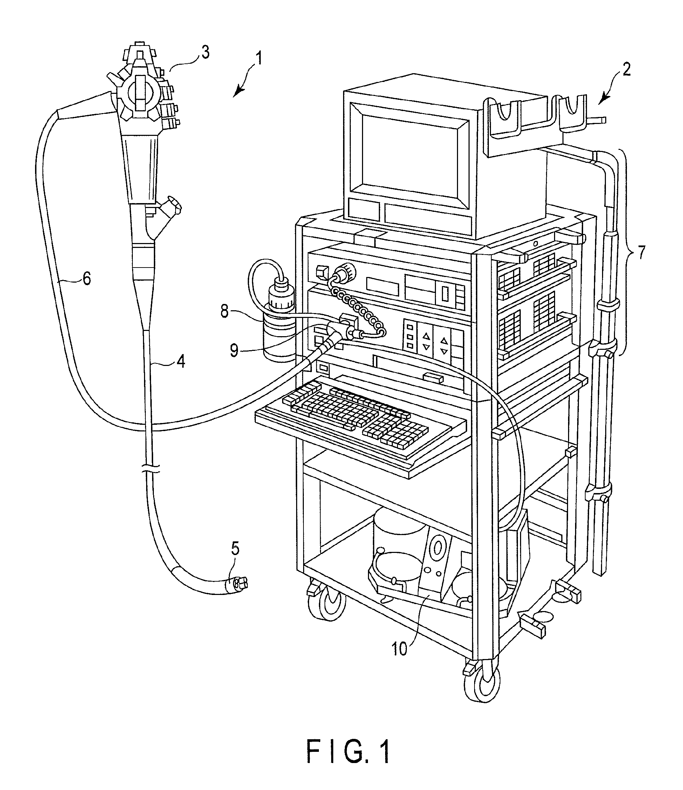

FIG. 1 is a view showing the external configuration of an endoscopic device provided with a cleaning mechanism configured to clean an imaging unit including a direct-viewing observation port, and side-viewing observation port according to a first embodiment.
The endoscopic device of this embodiment is roughly constituted of an endoscope main body 1, and endoscopic apparatus 7 mounted on a movable trolley 2. In the following description, although a description will be given by taking a flexible scope as an example, a rigid scope can also be provided.
The endoscope main body 1 is constituted of an insertion section (flexible tube) 4 to be inserted into a body cavity which is an observation object, bending section 5 which is provided on the tip side of the insertion section 4, and to which a lens unit to be described later is provided at the tip thereof, and operation section 3 configured to make the bending section 5 carry out a bending operation. In the following description, the bending section 5 side is referred to as the tip side, and the operation section 3 side is referred to as the proximal end side, while the insertion section 4 is regarded as the center.

The endoscopic apparatus 7 include a light-source unit configured to create illumination light used to illuminate the observation object part, video processor configured to subject an image signal acquired by imaging to predetermined image processing, monitor configured to display an image signal as an observation image, keyboard serving as an input section, and the like.

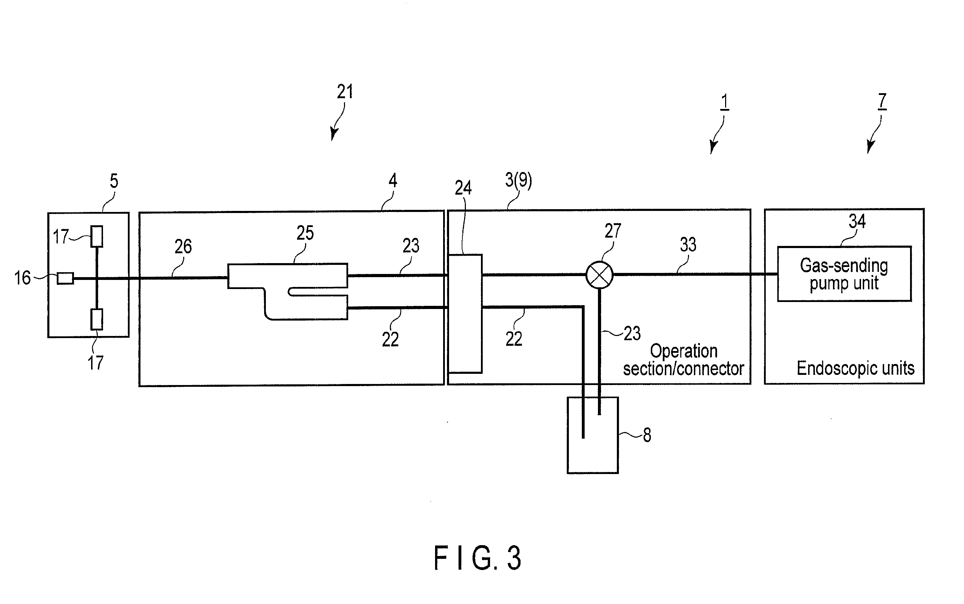

Furthermore, a bottle 8 used to retain a liquid (cleaning liquid: for example, a liquid mainly constituted of water such as physiological sodium chloride solution or the like) used for cleaning or the like is detachably attached to a supporting column of the trolley 2. Further, inside the endoscopic apparatus 7, a gas-sending pump unit 34 (see FIG. 3) is arranged. Furthermore, on a shelf of the trolley 2, a suction unit 10 configured to suck a liquid or gas gushed into the body cavity from a wash nozzle to be described later in the body cavity is provided.

The endoscope main body 1, and the light-source unit are connector-connected to each other with a universal cable 6. The universal cable 6 includes, in addition to a light guide constituted of an optical fiber, a plurality of signal lines configured to transmit an image signal, and the like, and supply routes (gas-sending/liquid-sending channels) and exhaust routes of a gaseous body and liquid body constituted of tubes. A connector of the universal cable 6 configured to connect the cable 6 to the endoscopic apparatus 7 side branches the cable 6 into the signal lines, tubes, and light guide, and connects these branched members to their corresponding configuration parts.

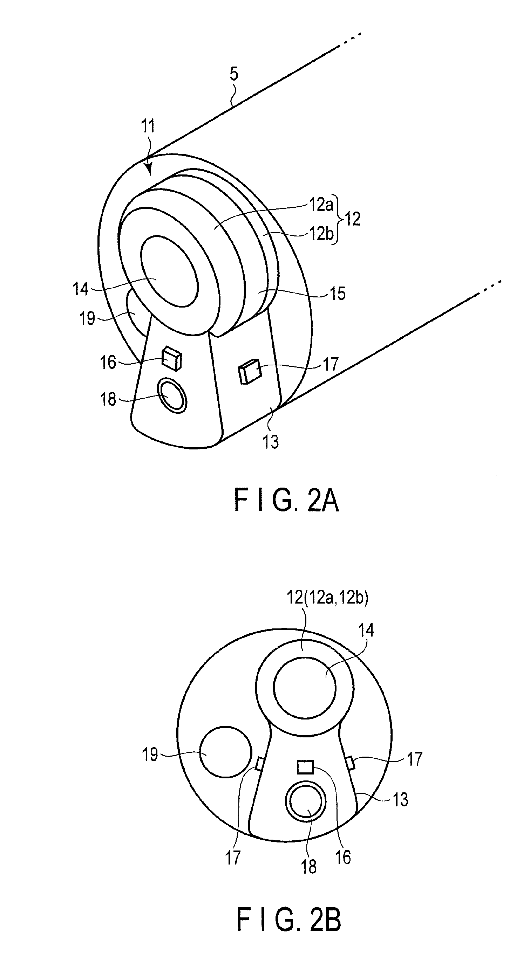

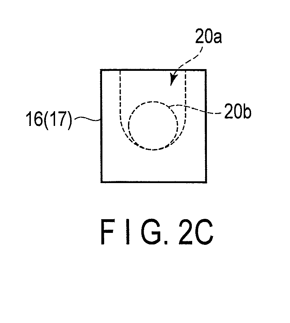

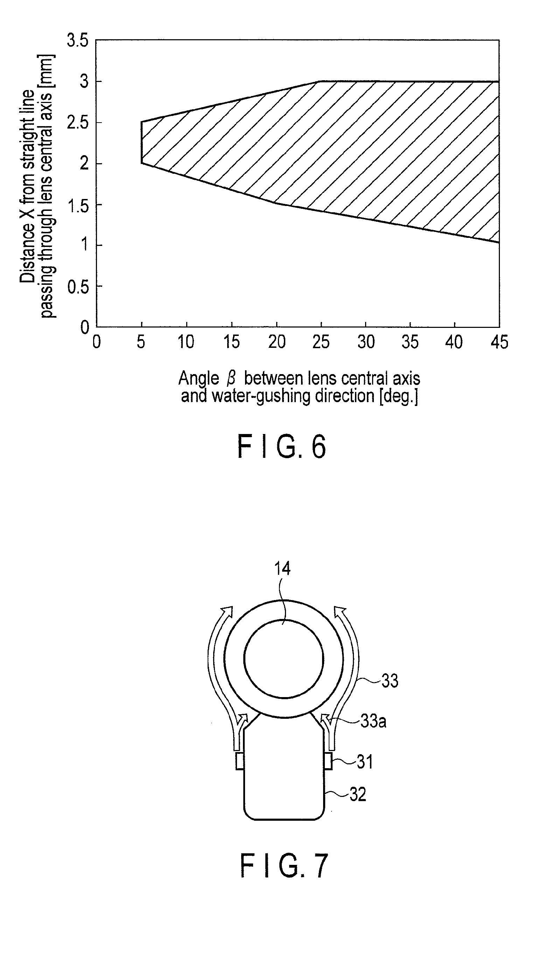

FIG. 2A is a view showing the external configuration of the insertion section tip. FIG. 2B is a view showing the configuration of the insertion section tip viewed from the front. FIG. 2C is a view showing a configuration example of a wash nozzle. FIG. 3 is a view showing a configuration example of a gas-sending/liquid-sending mechanism of this embodiment. FIG. 7 is a view conceptually showing the configuration of the optical system of the imaging section of the endoscope. In the following description, a direction of movement of the insertion section in the body cavity is referred to as an insertion direction or an axial direction, the face of the insertion section viewed from the axial direction is referred as the front face (tip face), and the face of the insertion section orthogonality to the axial direction thereof is referred to as a lateral face or a laterally circumferential face.

On the tip face of the bending section 5 of the insertion section, a lens unit 11 constituted of a direct-viewing observation port 14, and side-viewing observation port 15 to be described later, pedestal 13 which is a tip structure having a front face flush with the face of the direct-viewing observation port 14 (face having a height identical to the forward-protrusion height of the direct-viewing observation port 14), wash nozzle 16 arranged on the front face side of the pedestal 13 in close proximity to the direct-viewing observation port 14, two wash nozzles 17 arranged on both lateral faces of the pedestal 13, aperture section 19 of a forceps hole used to insert forceps (not shown) or the like, direct-viewing observation port 14 used to carry out imaging in the insertion direction (axial direction) in a predetermined visual field region, and illumination port 18 arranged on the front face of the pedestal 13, and used to apply illumination light for the direct-viewing observation port 14 are arranged.

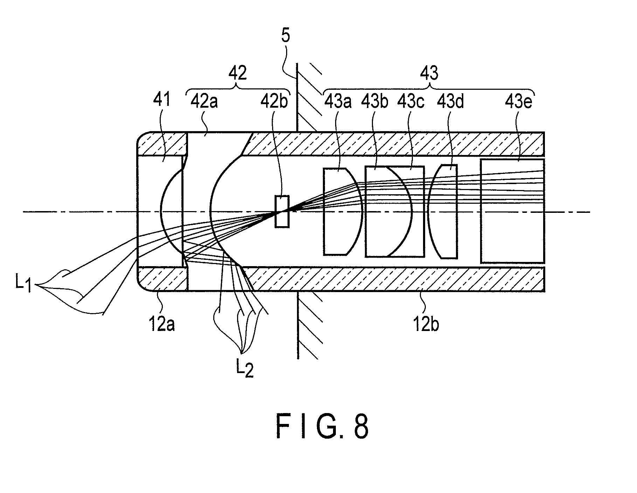

In the lens unit 11, the direct-viewing observation port 14 is arranged on the front face when viewed from the axial direction, and the side-viewing observation port 15 is arranged on the rear side (proximal end side) of the port 14. The lens unit 11 is a part of the imaging optical system of the endoscope as shown in FIG. 8, is arranged in front of an imaging element (not shown), and is constituted of a plurality of lenses, and lens groups, and lens frames (12a, 12b) 12 supporting the lenses in order to form a captured image.

In this example, the direct-viewing observation port 14 are constituted of a circular concave lens 41 (first lens), first lens group (42a, 42b) 42, and second lens group (43a, 43b, 43c, 43d, 43e) 43. The light flux L1 incident on the direct-viewing observation port 14 passes through the lenses 41, and 42a, is collected at the lens 42b serving as a focal point, then further passes through the second lens group, and is guided to the imaging element (not shown) as a light figure.

Further, the side-viewing observation port 15 is constituted of a cylindrical lens 42a. The cylindrical lens 42a is arranged in such a manner that the lateral face (referred to as a laterally circumferential face) of the circumference thereof is exposed, and is a lens the front side and back side of which are formed into a convex and concave. The light flux L2 incident on the laterally circumferential face from the lateral circumference is reflected from the concave, and is directed to the convex side. The light flux L2 further reflected from the convex surface passes through the concave, then is collected at the lens 42b serving as a focal point, then passes through the second lens group, and is further guided to the imaging element (not shown) as a light figure. By the lens configuration described above, an image or a constructed image based on each of direct viewing, and side viewing is created by utilizing the same imaging element.

Further, regarding both of or one of the cylindrical lens frames 12a, and 12b each having a cylindrical shape, the whole or part of the circumferential face corresponding to a place at which the cylindrical lens is exposed to the outside is formed of a light guiding member (translucent member). At least part of (not shown) the lens frame 12b guides the illumination light branched from the light guide. The guided illumination light is applied as the illumination light in the laterally circumferential direction to illuminate the observation visual field of the side-viewing observation port from each lens frame surface.

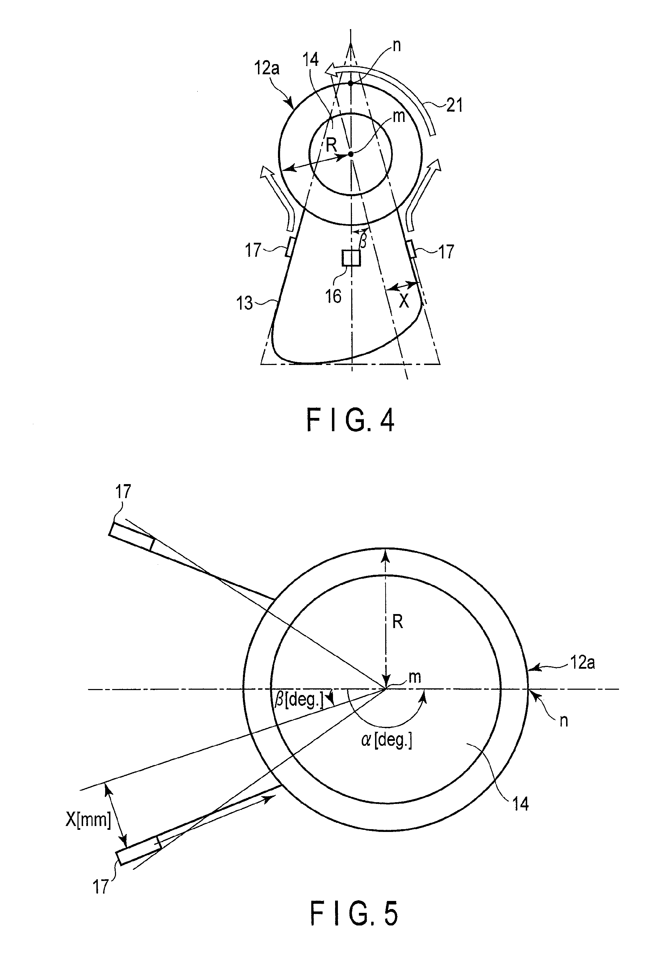

Inside the pedestal 13, a liquid-sending route, and gas-sending route to be connected to the wash nozzles are formed and, furthermore an optical fiber cable is arranged in order to guide the illumination light to the illumination port 18 arranged on the front face. As the shape of the pedestal 13, an approximately triangular shape as indicated by alternate long and short dash lines in FIG. 4 is assumed, the part which virtually becomes the base of the triangle becomes the circular arc surface identical to the circumferential surface of the tip of the bending section 5, and the parts which become the oblique sides of the triangle become the two lateral faces directed from both ends of the base to the central part of the direct-viewing observation port 14. At this central part, more specifically, a perpendicular line vertically passing through the center m of the direct-viewing observation port 14 is assumed, and assuming that the perpendicular line, and the base vertically intersect each other, the virtual intersection point of the two lateral faces is on the perpendicular line. Furthermore, the triangle, and the lens unit 11 overlap each other in such a manner that the crowning part of the triangle including the virtual intersection point of the two lateral faces is positioned within the direct-viewing observation port 14 and, substantially, the pedestal 13 is formed into approximately a fan shape adhering closely to the lens unit 11.

On each of the two lateral faces of the pedestal 13, a wash nozzle 17 is provided. A cleaning liquid gushed from the wash nozzle 17 flows in contact with the lateral face of the side-viewing observation port 15, and further flows to go around the top part of the lens unit 11. The side-viewing observation port 15 is cleaned by such a liquid current. As shown in FIG. 2C, in each of the wash nozzles 16, and 17, a face thereof facing the observation port is made the aperture face (ejection hole) 20a, and the inside thereof is made hollow. In the hollow part of the wash nozzle 16 or 17, an aperture 20b of the gas-sending/liquid-sending route is formed on the pedestal face. When cleaning is carried out, a cleaning liquid or a gaseous body gushed from the gas-sending/liquid-sending aperture 20b is gushed from the nozzle aperture face 20a, and is blown against each observation port.

FIG. 3 is a view showing a configuration example of a gas-sending/liquid-sending mechanism of this embodiment.

As shown in FIG. 1, and FIG. 3, in the gas-sending/liquid-sending mechanism 21, a liquid serving as the cleaning liquid is contained in a bottle 8, and the liquid is sent from the bottle 8 to the endoscope 1 through the tube, connector 9, and universal cable 6. Inside the insertion section of the endoscope 1, a liquid-sending tube (channel) 22, and gas-sending tube 23 are provided, and a confluence section 25 at which these tubes join each other through a cylinder section 24 provided in the operation section 3 is arranged. Furthermore, a gas-sending/liquid-sending tube 26 used to send the merged gas-supply/liquid-supply is provided on the tip side of the confluence section 25, and is then branched into at least three paths at a position near the tip section of the bending section, and the branched paths are connected to the wash nozzles 16, and 17. It should be noted that in order to enhance the moving speed of the fluid current gushed from the aperture 20b obtained by mixing the cleaning liquid, and gaseous body or of only the liquid or of only the gaseous body as the need arises, a throttle is provided to the aperture 20a or 20b.

Further, the gas-sending tube 23 connected to the cylinder section 24 is branched at a valve 27 arranged in the operation section 3, and the branched gas-sending tube 23 is inserted into the externally provided bottle 8. The gaseous body sent from the gas-sending pump unit 34 inside the endoscopic apparatus 7 can be switched to only the cylinder section 24 side, to only the bottle 8 side or to both the cylinder section 24 side, and the bottle 8 side. These switching operations are carried out in accordance with the operation of the cylinder section 24, and the cleaning liquid mixed with the gaseous body, only the cleaning liquid or only the gaseous body is selected by a button operation, and is gushed from the wash nozzles.

When air is used as the gaseous body, the gaseous body is sent from the gas-sending pump unit 34 into the inside of the endoscope through the connector 9, and universal cable 6. In the case where a gaseous body other than air is to be employed, a gas cylinder or the like is used.

The shape of the pedestal on which the wash nozzles used to clean the lens unit 11 are arranged will be described below with reference to FIGS. 4 to 7. FIG. 4 shows the state of the cleaning liquid gushed from the wash nozzle flowing with respect to the pedestal. FIG. 5 is a view showing the positional relationship between the wash nozzles, and lens unit 11. FIG. 6 is a view showing a relationship between a distance X from a straight line passing through the central axis of the lens, and angle $\beta$ between the lens central axis and water-gushing direction. FIG. 7 shows the state of the cleaning liquid gushed from the wash nozzles flowing with respect to the pedestal examined previously.

First, cleaning of the observation port using a cleaning liquid or a gaseous body will be described below.

It is ideal that in the side-viewing observation port 15 of the lens unit 11, the observation visual field range covers the entire circumference. However, as described previously, a pedestal is required to arrange a wash nozzle for each of the direct-viewing observation port 14, and side-viewing observation port 15. Thus, as shown in FIG. 7, an idea of arranging a pedestal beside the lens unit 11 in the shape of a keyhole is conceivable.

In this arrangement, when the rectangular pedestal 32 is made to abut on the side-viewing observation port 15, an idea of forming the keyhole shape in a constricted style by obliquely removing the corner of the part at which the pedestal 32 abuts on the side-viewing observation port 15 is conceivable in order to secure the observation visual field of the side-viewing observation port 15. In the case of a shape having such "constriction", wash nozzles 31 are to be arranged on the lateral faces parallel to each other. Each of the wash nozzles 31 on the parallel lateral faces is to gush the cleaning liquid 21 not in the direction to the center (position identical to the center of the direct-viewing observation port 14 in FIG. 4) of the side-viewing observation port 14, but in the tangential direction of the circumferential face of the observation port.

Accordingly, as shown in FIG. 7, although part of the flow of the cleaning liquid 33 gushed from each of the wash nozzles 31 flows into the inside of the constricted part, the branched flow cannot thereafter join the flow of the cleaning liquid 33 which is the mainstream, and a situation in which the part of the flow is retained at the constricted part occurs. That is, it is conceivable that a foreign substance adhering to the constricted part will become hardly removable. In general, it is known that dirt is liable to collect at a constricted part, and it is difficult to remove the collected dirt.

Although it is sufficient if a wash nozzle is arranged at the constricted part, it is difficult to arrange a liquid-sending route or a gas-sending route at the constricted part. Further, although it is also possible to make the flow of the cleaning liquid go around via the constricted part by providing an auxiliary member such as a rectification board or the like upstream from the constricted part, when such an auxiliary member is provided at the tip part of the insertion section to be inserted into the body cavity, a situation in which a foreign subject gets jammed in the constricted part to thereby hinder the flow of the cleaning liquid is also conceivable. Thus, arranging such an auxiliary member is not desirable.

In this embodiment, as shown in FIG. 4, the pedestal is formed into such a shape that the cleaning liquid gushed from the wash nozzle is immediately passed through the constricted part, and is then directed toward the circumferential face of the side-viewing observation port 15 by forming the lateral face on which the wash nozzle is to be arranged in an oblique direction, i.e., in a direction to the center of the side-viewing observation port 15. Here, the gushing angle, and the gushing position of the wash nozzle that make the gushed cleaning liquid flow along the circumferential face of the side-viewing observation port 15 become important.

Thus, as an examination example shown in FIG. 5, it is assumed that the diameter 2R of the cylindrical lens of the side-viewing observation port 15 is $\varphi$6.2 mm, and the amount of the cleaning liquid to be gushed is 37.5 mL. Further, FIG. 5 is shown by assuming that the lens diameter 2R of the side-viewing observation port 15 is identical to the diameter of the lens frame 12. As the angle $\beta$ becomes larger, the size of the pedestal 13 becomes larger, and hence the visual field from the side-viewing observation port 15 is largely interrupted. For this reason, the angle $\beta$ is made smaller than or equal to 45°. When the angle $\beta$ exceeds 45°, the cleaning liquid 21 begins not to flow uniformly along the circumferential face of the lens. Further, when the angle $\alpha$ is greater than or equal to 180°, the cleaning liquid 21 can sufficiently go around via the top n. That is, the cleaning liquid can travel along the entire circumferential face of the lens. The result of the experiment carried out in this examination example is shown in FIG. 6. Here, the X-axis indicates the angle $\beta$ shown in FIG. 5, and the Y-axis indicates the distance X shown in FIG. 5. By this examination, the following result has been obtained. The angle $\beta$ is greater than or equal to 5°, and smaller than or equal to 45°, and desirably smaller than or equal to 30°. The distance X from an oblique line passing through the central point of the lens having an angle of $\beta$ to the lateral face is 1mm to 3mm, and desirably 1.5mm to 3mm. That is, a result indicating that a distance corresponding to 1 to 1/2 of the radius R of the lens is desirable has been obtained. It goes without saying that in this examination example, in the detailed part, a somewhat different result is conceivable depending on the lens diameter, and the amount of the liquid to be sent, and hence it is desirable that each of the detailed matters be verified at the time of design.

Hereinafter, items which provide results and tendencies that are universal to all the cases even when different lens diameters and amounts of the cleaning liquid to be sent are employed will be described below.

    a. The lateral faces are arranged oblique in such a manner that the direction of the cleaning liquid gushed from the aperture face (ejection hole) of each of two wash nozzles forms an acute angle greater than or equal to 5°, and smaller than or equal to 45° with a straight line passing through the center (center m of the side-viewing observation port 15) around which the flow of water is made to go along the circumferential face of the observation port 15. Accordingly, the angle between the gushing directions of the cleaning liquid gushed from the two wash nozzles intersecting each other is about 10 to 90°.

    b. When the lateral face (nozzle arrangement face) of the pedestal has an inclination of, for example, 45° or less, the first straight line along the lateral face, and the second straight line passing through the center m of the lens, and having an inclination identical to the inclination of the lateral face are parallel to each other, and the distance between the first and second straight lines is X, and the radius of the lens is R, the following relationship is obtained.

    $1/2R \leq X < R$

According to this embodiment described above, it is possible to secure a space in which a liquid-sending route, gas-sending route, and various parts are to be arranged, and carry out cleaning in such a manner that a foreign substance adhering to the faces of the observation ports including the joint part between the cylindrical lens serving as the side-viewing observation port, and pedestal is efficiently and completely removed.

Next, a second embodiment will be described below.

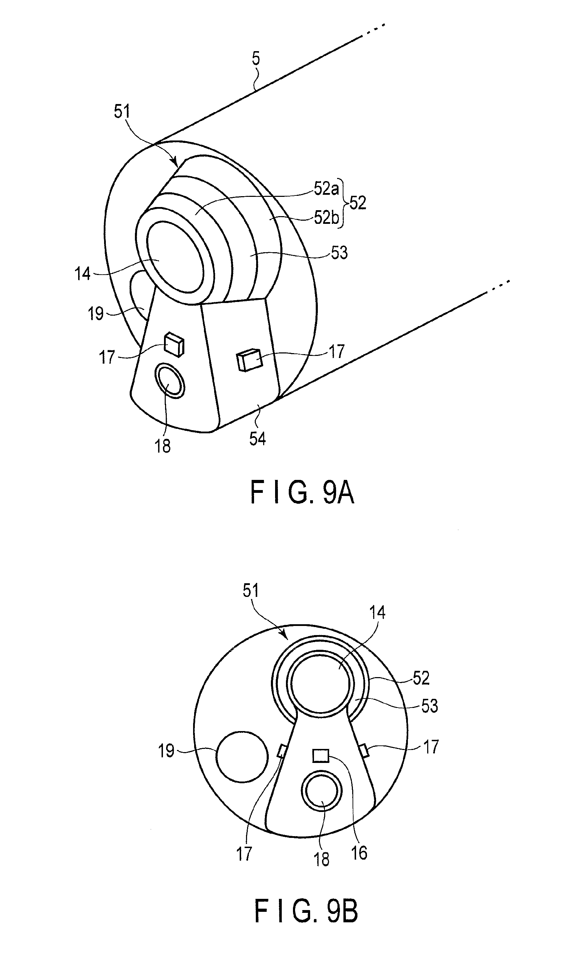

FIG. 9A is a view showing the external configuration of an insertion section tip of an endoscopic device according to the second embodiment, and FIG. 9B is a view showing the configuration of the insertion section tip viewed from the front. It should be noted that in the configuration parts of this embodiment, configuration parts identical to the configuration parts of the first embodiment described previously are denoted by identical reference symbols and their detailed description are omitted.

A lens unit 51 of this embodiment has a shape of a circular truncated cone, whereas the lens unit 11 in the first embodiment is cylindrical.

As shown in FIG. 9A, the lens unit 51 is constituted by laying a lens frame 52a, truncated cone lens 53, and lens frame 52b one on top of the other, and is formed into a shape of a circular truncated cone smoothly tapered off. On the tip face which is the top face of the circular truncated cone, a direct-viewing observation port 14 is arranged.

A pedestal section 54 is joined to the lens unit 51 in such a manner that the joint part between the pedestal section 54, and lens unit 51 forms oblique lines directed toward the direct-viewing observation port 14 side. Further, as in the first embodiment, in both of or one of the lens frames 52a, and 52b, the whole or part of the circumferential face corresponding to a place at which the lens is exposed to the outside is formed of a translucent member.

The lens frames 52a, and 52b guide the illumination light branched from the light guide, and applies the illumination light in the laterally circumferential direction from the lens frame surfaces.

According to this embodiment, a function, and advantage identical to the first embodiment are produced. The joint part between the side-viewing observation port 53, and pedestal section 54 is in an oblique direction with respect to the direction in which the cleaning liquid or the gaseous body is gushed, and hence when the insertion section is moved, a foreign substance adhering to the insertion section moves in flux, and becomes hardly sticky. Furthermore, the gushed cleaning liquid or gaseous body continuously flows along the joint part in the oblique direction toward the tip side or the rear end side, and hence cleaning can be carried out by removing the dirt or the like more thoroughly.

Reference Signs List

1...endoscope main body, 2...trolley, 3...operation section, 4...insertion section (flexible tube), 5...bending section, 6...universal cable, 7...endoscopic apparatus, 8...bottle, 9...connector, 10...suction unit, 11...lens unit, 12, 12a, 12b, 52, 52a, 52b...lens frame, 13, 54...pedestal, 14...direct-viewing observation port, 15, 53...side-viewing observation port, 16, 17, 21...wash nozzle, 18...illumination port, 19...aperture section, 20a...nozzle aperture face, 20b, 20c...aperture, 34...gas-sending pump unit, 41...circular concave lens (cylindrical lens), 42, 42a, 42b...first lens group, 43, 43a, 43b, 43c, 43d, 43e...second lens group.

**Claims**

1. An endoscopic device including a cleaning mechanism **characterized by** comprising:

a cylindrical lens arranged at a tip of an insertion section of an endoscope as an optical member for observation with a face thereof laterally circumferential with respect to an axial direction which is an insertion direction exposed to the outside;
a pedestal section two lateral faces of which obliquely and straightly extending toward the central side of the cylindrical lens are joined to the laterally circumferential face, and inside which a liquid-sending route configured to send a cleaning liquid, and a gas-sending route configured to send a gaseous body are internally provided; and
two wash nozzles each of which is provided on each of the two lateral faces, and is configured to selectively gush a fluid current obtained by mixing the cleaning liquid, and the gaseous body, only the liquid or only the gaseous body,
an angle which gushing directions of the cleaning liquid straightly gushed from the two wash nozzles toward the laterally circumferential faces form with each other is an acute angle, the gushing directions intersecting each other, the cleaning liquid flows along the laterally circumferential face via the joint part between the pedestal section, and the laterally circumferential face.

2. The endoscopic device according to claim 1, **characterized in that** the wash nozzles are arranged on both the lateral faces in such a manner that the gushing direction of the cleaning liquid straightly gushed from each of the wash nozzles forms an angle greater than or equal to 5°, and smaller than or equal to 30° with a straight line passing through a center of the cylindrical lens in a radial direction.

3. The endoscopic device according to claim 1, **characterized in that** a distance X between a first straight line along one of the lateral faces of the pedestal section, and a second straight line passing through the center of the cylindrical lens with an inclination identical to the inclination of the first straight line, and parallel to the first straight line, and a lens radius R of the cylindrical lens satisfies the following relationship.

$$1/2R \leq X < R$$

4. The endoscopic device according to claim 1, **characterized in that** a lens group including the cylindrical lens provided at the tip of the insertion section constitutes a side-viewing observation port configured to make a light figure of an observation object incident on the laterally circumferential face, and
furthermore, a first lens arranged on the front side of the cylindrical lens at the tip of the insertion section in a superposing manner constitutes a direct-viewing observation port configured to make a light figure of an observation object in the axial direction incident thereon.

5. The endoscopic device according to claim 4, **characterized in that** the side-viewing observation port, and the direct-viewing observation port assume an external appearance of a cylindrical shape.

6. The endoscopic device according to claim 4, **characterized in that** the side-viewing observation port, and the direct-viewing observation port assume an external appearance of a circular truncated cone.

7. The endoscopic device according to claim 4, **characterized in that** at least part of a lens frame configured to support the lens group of the side-viewing observation port, and the first lens of the direct-viewing observation port is formed of a light guiding member configured to guide illumination light, and irradiates an observation visual field of the side-viewing observation port with illumination light.

F I G. 1

FIG. 2A

FIG. 2B

20a

16(17)

20b

# F I G. 2C

F I G. 3

12

EP 2 901 913 A1

F I G. 4

F I G. 5

F I G. 6

F I G. 7

F I G. 8

F I G. 9A

F I G. 9B

**INTERNATIONAL SEARCH REPORT**

International application No.

PCT/JP2013/067543

A. CLASSIFICATION OF SUBJECT MATTER

*A61B1/00*(2006.01)i, *G02B23/24*(2006.01)i

According to International Patent Classification (IPC) or to both national classification and IPC

B. FIELDS SEARCHED

Minimum documentation searched (classification system followed by classification symbols)
A61B1/00, G02B23/24

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2011/055641 A1 (Olympus Medical Systems Corp.),<br>12 May 2011 (12.05.2011),<br>paragraphs [0013], [0028] to [0034]; fig. 7, 8<br>& US 2011/0282148 A1 & EP 2420178 A1<br>& WO 2011/055641 A1 & CN 102469924 A | 1-4,5<br>7<br>6 |
| Y<br>A | WO 2011/055640 A1 (Olympus Medical Systems Corp.),<br>12 May 2011 (12.05.2011),<br>paragraph [0014]<br>& US 2011/0282155 A1 & US 2013/0109917 A<br>& EP 2380483 A1 & WO 2011/055640 A1<br>& CN 102341028 A | 7<br>6 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 July, 2013 (24.07.13) | 06 August, 2013 (06.08.13) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
|---|
| PCT/JP2013/067543 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2000-10022 A  (Fuji Photo Optical Co., Ltd.), 14 January 2000 (14.01.2000), fig. 1, 8 & US 6142932 A | 6 |

Form PCT/ISA/210 (continuation of second sheet) (July 2009)

## INTERNATIONAL SEARCH REPORT

International application No.

PCT/JP2013/067543

**Box No. II        Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:
    The invention in claim 1 and the invention in claim 3 have a common technical feature of "an endoscope device including a washing mechanism having a pedestal part and a cylindrical lens".
    However, the above-said technical feature cannot be considered to be a special technical feature, since the technical feature does not make a contribution over the prior art in the light of the contents disclosed in the document 1 (WO 2011/055641 A1 (Olympus Medical Systems Corp.)).
    Further, there is no other same or corresponding special technical feature between these inventions.
(Continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2013/067543

Continuation of Box No.III of continuation of first sheet(2)

Therefore, the inventions (invention groups), which are configured from invention 1 comprising claims 1, 2 and 4-7, and invention 2 comprising claim 3, are involved in claims.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010169792 A
- JP 2009015252 A

- JP 5103752 A